# EUROPEAN PATENT APPLICATION

(11) **EP 4 847 532 A1**
(43) Date of publication of application: **02.12.2026**
(21) Application number: 25745275.5
(22) Date of filing: 17.01.2025
(51) Int. Cl.: C07K 16/28, C12N 5/12, A61P 37/06, A61K 39/00

(54) **ANTIBODY SPECIFICALLY BINDING TO CD40 AND USE THEREOF**

(30) Priority: 24.01.2024 KR 20240010670
(71) Applicant: Seoul National University R&DB Foundation, Seoul 08826 (KR)
(72) Inventor: PARK, Chung Gyu, Seoul 06518 (KR); KIM, Jung Sik, Uiwang-si Gyeonggi-do 16017 (KR)
(74) Representative: Reddie & Grose LLP
(86) International application number: PCT/KR2025/099085
(87) International publication number: WO 2025/159610

(57) **Abstract**

The present invention relates to a novel anti-CD40 antibody and a use thereof, and more particularly, to a novel anti-CD40 antibody which comprises complementarity determining regions of specific sequences and which blocks CD40-CD40L signaling to exhibit an excellent antagonistic effect, and a use thereof. The novel anti-CD40 antibody of the present invention blocks CD40-CD40L signaling to exhibit an excellent antagonistic effect, and thus can be widely utilized as an agent for preventing or treating various diseases, such as autoimmune diseases and chronic inflammatory diseases, and as an agent for suppressing immune rejection upon transplantation.

## Description

### [TECHNICAL FIELD]

The present invention relates to a novel anti-CD40 antibody and a use thereof, and more particularly, to a novel anti-CD40 antibody which comprises complementarity determining regions of specific sequences and which blocks CD40-CD40L signaling to exhibit an excellent antagonistic effect, and a use thereof.

### [BACKGROUND ART]

The CD40 signal transduction pathway is governed by the combined regulation of many intracellular factors. Like other members of the TNF receptor family, CD40, upon binding of CD40 and CD40L (either solid-phase CD40L or soluble CD40L), interacts with TRAF proteins (TNF receptor-associated factors), such as TRAF2 and TRAF3, which mediate intracellular signals. TRAFs transduce the signal to the nucleus via MAP kinases, such as NIK (NF-κB-inducing kinase) and I-kappa B kinase (IKK α/β), thereby ultimately activating the transcription factor NF-κB (Young et al. (1998) Immunol Today 19:502-06). Signaling via the Ras and MEK/ERK pathways has also been demonstrated in a subset of B cells. Additional pathways involved in CD40 cell signaling include the PI3K/Akt pathway and the P38 MAPK pathway (Craxton et al. (1998) J Immunol 5:439-447).

Signaling via CD40 has been shown to prevent cell death from apoptosis (Makus et al. (2002) J Immunol 14:973-982). Apoptotic signals are required to induce programmed cell death in a coordinated manner. Cell death signals may include intrinsic stimuli arising from within the cell, such as endoplasmic reticulum stress, or extrinsic stimuli, such as receptor binding of FasL or TNFα. The signaling pathway is complex and includes the activation of caspases, such as caspases 3 and 9, and the activation of poly(ADP-ribose) polymerase (PARP). During the cascade, anti-apoptotic signaling proteins, such as Mcl-1 and BCLx, and members of the IAP-family proteins, such as the X-linked inhibitor of apoptosis (XIAP), are down-regulated (Budihardjo et al. (1999) Annu Rev Cell Dev Biol 15:269-90). For example, CD40 cell signaling in dendritic cells can block the apoptotic signal transduced by FasL (Bjorck et al. (1997) Int'l Immunol 9:365-372).

Therefore, CD40 binding by CD40L and the subsequent activation of CD40 signaling are essential steps for a normal immune response, and dysregulation of the function of such CD40 signaling can cause autoimmune diseases (Ichikawa et al. (2002), J Immunol 169:2781-7 and Moore et al. (2002) J Autoimmun 19:139-45). In addition, the CD40/CD40L interaction also plays an important role in inflammatory processes. For example, both CD40 and CD40L are overexpressed in human and experimental atherosclerotic lesions. CD40 stimulation induces the expression of matrix-degrading enzymes and the expression of tissue factor in atheroma-associated cell types, such as endothelial cells, smooth muscle cells, and macrophages. Furthermore, CD40 stimulation induces the production of pro-inflammatory cytokines, such as IL-1, IL-6, and IL-8, and adhesion molecules, such as ICAM-1, E-selectin, and VCAM. Inhibition of the CD40/CD40L interaction prevents atherogenesis in animal models.

CD40 is a 55 kDa cell-surface antigen that appears on the surface of normal and neoplastic human B cells, dendritic cells, antigen-presenting cells (APCs), endothelial cells, monocytes, CD8+ T cells, and epithelial cells. The CD40 antigen is also expressed on activated T cells, activated platelets, inflamed vascular smooth muscle cells, eosinophils, the synovium in rheumatoid arthritis, dermal fibroblasts, and other non-lymphoid cell types. Depending on the type of cell expressing CD40, ligation can induce intracellular adhesion, differentiation, activation, and proliferation. For example, binding to CD40L (also designated CD154), the ligand of CD40, stimulates B-cell proliferation and differentiation into plasma cells, antibody production, isotype switching, and B-cell memory regeneration. During B-cell differentiation, CD40 is expressed on precursor B cells but is lost upon differentiation into plasma cells. CD40 is involved in the differentiation, survival, and proliferation of B-lymphocytes, macrophages, antigen-presenting cells, and some endothelial cells and epithelial cells. CD40-CD154 signaling is known to be involved in isotype switching and affinity maturation in antibody production by B cells, and to be involved in T-cell activity in T cells (Leukoc Biol 67:2-17, 2000).

Although the CD40 ligand is identified on the cell surface of activated T cells (Fenslow et al. (1992) J Immunol 149:655; Lane et al. (1992) Eur J Immunol 22:2573; Noelle et al. (1992) Proc Natl Acad Sci USA 89:6550), it is generally not expressed on resting human T cells. CD40L is a type II transmembrane glycoprotein having homology with TNF-α (Armitage et al. (1992) Nature 357:80 and Spriggs et al. (1992) J Exp Med 176:1543). The extracellular domain of CD40L contains two arginine residues near the transmembrane region, which provide a potential proteolytic cleavage site that gives rise to a soluble form of the ligand (sCD40L). Overexpression of CD40L causes an autoimmune disease similar to systemic lupus erythematosus in rodent models (Higuchi et al. (2002) J Immunol 168:9-12). In contrast, the absence of functional CD40L on activated T cells causes X-linked hyper-IgM syndrome (Allen et al. (1993) Science 259:990; and Korthauer et al. (1993) Nature 361:539). Taken together, CD40-CD40L signaling is involved in the maturation of dendritic cells, secretion of inflammatory cytokines, and increased survival; is involved in B-cell activation, isotype switching, and antibody production; and is involved in macrophage activation and the increase of inflammatory cytokines. Accordingly, it is involved in the activation, proliferation, and differentiation of antigen-specific T cells (Jodi L. Karnell et al., 2019, Advanced Drug Delivery Reviews, vol. 141, 92-103). CD40-CD40L signaling enhances the cytotoxic function of NK cells (G. Terrazzano et al., Scand. J. Immunol., 59(4), 2004, 356-362). In addition, CD40 signaling promotes the survival of granulocytes and the secretion of GM-CSF (Y. Ohkawara et al., J. Clin. Invest., 97(7) (1996), 1761-1766).

CD40-CD40L signaling is central to the onset of autoimmune diseases and inflammatory diseases caused by autoantibodies (Jodi L. Karnell et al., 2019, Advanced Drug Delivery Reviews, vol. 141, 92-103).

CD40L is expressed not only on emerging follicular helper T (Tfh) cells in lymphoid tissues but also in germinal centers located in tissue ectopic follicles, and is believed to play a major role in the generation of plasma cells having autoimmune specificity. CD40-CD40L signaling is known to play a pivotal role in the production of pathogenic autoantibodies in systemic rheumatic diseases, such as SLE, lupus nephritis, Sjögren's syndrome, myositis, systemic sclerosis, ANCA vasculitis, rheumatoid arthritis, IgG4-related disease, and pemphigus, in which autoantibodies are known to play an important role in disease progression.

CD40 signaling is also known to be essential for the production of autoantibodies against non-rheumatic diseases having a large autoantibody component, such as myasthenia gravis, neuromyelitis, NMDA receptor encephalitis, Hashimoto's thyroiditis or Graves' disease, idiopathic thrombocytopenic purpura, hemolytic anemia, or IgA nephropathy.

Therefore, CD40-CD40L signaling is involved in the onset of various forms of autoimmune diseases. Representative examples thereof are as follows: rheumatoid arthritis (M. Harigai et al., J. Rheumatol., 26(5) (1999), pp. 1035-1043), Sjögren's syndrome (S. Beaudreuil, Ann Transl Med, 3(9) (2015), p. 115), autoimmune nephritis (S. Doublier et al., PLoS One, 12(11) (2017)), autoimmune skin diseases such as systemic sclerosis (M. Caproni et al., J. Rheumatol., 34(12) (2007), pp. 2412-2416), multiple sclerosis (D. Chen et al., J. Immunol., 197(11) (2016), pp. 4257-4265), and type 1 diabetes (B. Balasa et al., J. Immunol., 159(9) (1997), pp. 4620-4627).

Blocking CD40-CD40L signaling inhibits the production of anti-drug antibodies and is also important for the treatment of inflammatory diseases such as inflammatory bowel disease (S. Danese et al., J. Immunol., 176(4) (2006), pp. 2617-2624).

Blocking CD40-CD40L signaling is absolutely critical for the suppression of rejection and the long-term survival of grafts in allogeneic and xenogeneic islet transplantation. In an allogeneic islet transplantation model using primates, both anti-CD154 antibodies and anti-CD40 antibodies are known to induce long-term survival of transplanted islets through mechanisms of suppressing antibody production by B cells and suppressing activation of T cells (Am J Transplant. 2012 Aug;12(8):2079-87).

However, paradoxically, the immunosuppressants currently used in autoimmune diseases and allogeneic islet transplantation cause various side effects in recipients upon long-term use. Therefore, there is a need for the development of novel immunosuppressants (or immunosuppressive antibodies) that have fewer side effects and are specific to particular immune cells.

That is, in autoimmune diseases, inflammatory diseases, and cell/organ transplantation, both anti-CD154 antibodies and anti-CD40 antibodies can treat immune diseases through mechanisms of suppressing antibody production by B cells and suppressing activation of T cells, and induce long-term survival of transplanted cells/organs. Blocking CD40-CD154 signaling in such immune cells is the core of immunosuppressive therapy, and blocking the interaction of CD40/CD40L can prevent transplant rejection and treat autoimmune diseases.

Korean Patent Application Publication No. 10-2021-0093968 discloses an antagonistic CD40 monoclonal antibody that binds to CD40 and does not exhibit CD40 agonist activity, and a use thereof; Korean Patent Application Publication No. 10-2019-0028508 discloses a multispecific antibody that binds to human CD40 and human CD137; and Korean Patent Registration No. 10-2198998 discloses an anti-CD40 antibody which directly targets CD40 rather than the CD40 ligand and which, without stimulating platelets, blocks CD40-CD154 signaling to exhibit an excellent antagonistic effect, and a use thereof.

Nevertheless, there is still a need for the development of a therapeutically and clinically relevant anti-CD40 antibody as a substance capable of effectively inhibiting the interaction of CD40/CD40L.

### [SUMMARY OF INVENTION]

### [TECHNICAL PROBLEM]

Under this background, the present inventors made intensive efforts to develop an antibody that blocks CD40-CD40L signaling, and as a result, completed the present invention by confirming that a novel anti-CD40 antibody targets CD40 to block CD40-CD40L signaling, thereby exhibiting an excellent antagonistic effect.

One object of the present invention is to provide an anti-CD40 antibody or an antigen-binding fragment thereof comprising the following complementarity determining regions (CDRs): (i) an HCDR1 (heavy chain complementarity determining region 1) having the amino acid sequence of SEQ ID NO: 1 or a sequence having at least 80% sequence identity thereto; (ii) an HCDR2 having the amino acid sequence of SEQ ID NO: 2 or a sequence having at least 80% sequence identity thereto; (iii) an HCDR3 having the amino acid sequence of SEQ ID NO: 3 or a sequence having at least 80% sequence identity thereto; (iv) an LCDR1 (light chain complementarity determining region 1) having the amino acid sequence of SEQ ID NO: 4 or a sequence having at least 80% sequence identity thereto; (v) an LCDR2 having the amino acid sequence of SEQ ID NO: 5 or a sequence having at least 80% sequence identity thereto; and (vi) an LCDR3 having the amino acid sequence of SEQ ID NO: 6 or a sequence having at least 80% sequence identity thereto.

Another object of the present invention is to provide a nucleic acid encoding the anti-CD40 antibody or antigen-binding fragment thereof.

Another object of the present invention is to provide a vector comprising the nucleic acid.

Another object of the present invention is to provide a host cell comprising the nucleic acid.

Another object of the present invention is to provide a method for producing an antibody, comprising culturing the host cell to express the antibody.

Another object of the present invention is to provide an antibody-drug conjugate comprising the anti-CD40 antibody or antigen-binding fragment thereof as an active ingredient.

Another object of the present invention is to provide a pharmaceutical composition for preventing or treating an autoimmune disease, comprising the antibody or antigen-binding fragment thereof as an active ingredient.

Another object of the present invention is to provide a pharmaceutical composition for preventing or treating a chronic inflammatory disease, comprising the antibody or antigen-binding fragment thereof as an active ingredient.

Another object of the present invention is to provide a composition for suppressing immune rejection upon transplantation, comprising the antibody or antigen-binding fragment thereof as an active ingredient.

Another object of the present invention is to provide a composition for reducing or preventing the formation of an anti-drug antibody (ADA) against a therapeutic agent, comprising the antibody or antigen-binding fragment thereof as an active ingredient.

### [SOLUTION TO PROBLEM]

Unless otherwise defined, the scientific and technical terms used in connection with the present invention shall have the meanings that are commonly understood by those of ordinary skill in the art. In addition, unless otherwise required by context, singular terms shall include pluralities and plural terms shall include the singular. Generally, the nomenclature and techniques used in connection with the cell and tissue culture, molecular biology, immunology, microbiology, genetics, and protein and nucleic acid chemistry and hybridization disclosed herein are those well known and commonly used in the art.

In order to achieve the above object, one embodiment of the present invention provides an anti-CD40 antibody or an antigen-binding fragment thereof comprising the following complementarity determining regions (CDRs): (i) an HCDR1 (heavy chain complementarity determining region 1) having the amino acid sequence of SEQ ID NO: 1 or a sequence having at least 80% sequence identity thereto; (ii) an HCDR2 having the amino acid sequence of SEQ ID NO: 2 or a sequence having at least 80% (preferably at least 85%, more preferably at least 90%, even more preferably at least 95%, or most preferably at least 99%; the same shall apply hereinafter to portions related to sequence identity) sequence identity thereto; (iii) an HCDR3 having the amino acid sequence of SEQ ID NO: 3 or a sequence having at least 80% sequence identity thereto; (iv) an LCDR1 (light chain complementarity determining region 1) having the amino acid sequence of SEQ ID NO: 4 or a sequence having at least 80% sequence identity thereto; (v) an LCDR2 having the amino acid sequence of SEQ ID NO: 5 or a sequence having at least 80% sequence identity thereto; and (vi) an LCDR3 having the amino acid sequence of SEQ ID NO: 6 or a sequence having at least 80% sequence identity thereto.

As used herein, the term "antibody" refers to a protein that binds to another molecule (antigen) through the variable regions of a light chain and a heavy chain, and includes the IgG, IgD, IgA, IgE, and IgM types. Antibodies include polyclonal antibodies, monoclonal antibodies, and multispecific antibodies. In addition, the antibody herein is intended to include monoclonal antibodies having structures of various forms, for example, an intact antibody (intact Ab) comprising two full-length heavy chains and two full-length light chains, as well as fragments thereof with or without a constant region, a chimeric antibody, a human antibody, a humanized antibody, or other genetically engineered antibodies having the characteristics according to the present application.

As used herein, the term "antigen-binding fragment" refers to a portion of the above-described intact antibody, and is a sequence that is shorter, in terms of length, than the amino acid sequence of the intact antibody by one or more sequences. In terms of function, it comprises at least a part of the activity or function of the intact antibody or the parent antibody, and examples thereof include, but are not limited to,

Fab (Fragment for antigen binding), Fab', F(ab')2, Fv, or a single-chain antibody (Single Chain Antibody, SCA) (e.g., scFv or dsFv), a bispecific scFv, and a diabody.

As used herein, the term "variable region" refers to an antigen-binding site formed by a portion of the heavy chain and the light chain, and each variable region consists of four sequence-conserved frameworks (FRs) and three highly sequence-variable complementarity determining regions (CDRs, Complementarity Determining Regions). The CDRs of the immunoglobulin heavy chain variable region (VH) are referred to as HCDR1 to HCDR3, and the CDRs of the immunoglobulin light chain variable region (VL) are referred to as LCDR1 to LCDR3.

As used herein, the term "complementarity determining region" refers to a site that determines the specificity and binding strength of an antibody for an antigen, and is where the most sequence variation between antibodies is found. Among these, the variation in the CDR3 site is the most severe, and it is composed of amino acid residues ranging from as few as 2 amino acids to as many as 26 or more. The portions of VH and VL other than the CDRs are framework residues. The framework of the antigen-binding polypeptide herein may use, as a consensus sequence, sequences found in naturally occurring human antibodies or sequences found in various antibodies.

The antibody according to the present application includes antigen-binding fragments, variants, or derivatives thereof. The antibody according to the present application may be of any type, for example, IgG, IgE, IgM, IgD, IgA, or IgY, and may also be of any class, for example, IgG1, IgG2, IgG3, IgG4, IgA1, or IgA2, or may be a subclass thereof.

In the present invention, the anti-CD40 antibody may comprise a heavy chain variable region having the amino acid sequence of SEQ ID NO: 13 or a sequence having at least 80% sequence identity thereto, and a light chain variable region having the amino acid sequence of SEQ ID NO: 14 or a sequence having at least 80% sequence identity thereto.

The present invention includes those in which a conservative substitution has occurred in the amino acids of SEQ ID NOs: 1 to 8. In the present invention, the substitution has occurred in fewer than 10, fewer than 9, fewer than 8, fewer than 7, fewer than 6, fewer than 5, fewer than 4, fewer than 3, fewer than 2, or 1 amino acid.

As used herein, the term "conservative substitution" is a term widely used in the art that refers to the substitution of one amino acid with another amino acid of similar characteristics. Similar characteristics include, for example, size, hydrophobicity, or charge. Amino acids are typically classified, according to the electrical characteristics of their side chains, into amino acids having a positively charged side chain, a negatively charged side chain, an uncharged side chain, or a hydrophobic side chain. For example, conservative substitutions include the substitution of leucine (Leu) with isoleucine (Ile), arginine (Arg) with lysine (Lys), phenylalanine (Phe) with tryptophan (Trp), aspartic acid (Asp) with glutamic acid (Glu), or serine (Ser) with threonine (Thr), or vice versa. In general, a conservative substitution in a CDR sequence does not essentially affect the function of the CDR.

Such a method for substituting sequences is known in the art, and reference may be made, for example, to Sambrook, Molecular Cloning: A Laboratory Manual (Fourth Edition), Cold Spring Harbor Laboratory (2012) NY.

In the present invention, the anti-CD40 antibody or antigen-binding fragment thereof may be produced by a hybridoma cell line deposited under accession number KCLRF-BP-00523.

In the present invention, the anti-CD40 antibody may be selected from the group consisting of a monoclonal antibody, a chimeric antibody, a primatized antibody, a humanized antibody, and a human antibody, but is not limited thereto.

The anti-CD40 antibody or antigen-binding fragment thereof herein may be a chimeric antibody. As used herein, the term "chimeric antibody" refers to an antibody in which at least a part of the variable region, i.e., the antigen-binding site, and the constant region of the antibody (including the CL1 region in the case of the light chain, and the CH1, CH2, and CH3 regions in the case of the heavy chain) is derived from different species. For example, the variable region may be of mouse origin, and the constant region may include one of human origin. Alternatively, it also refers to a class-switched antibody, for example, an antibody switched from the IgG type to the IgE type.

In addition, the anti-CD40 antibody or antigen-binding fragment thereof herein may be a humanized antibody. As used herein, the term "humanized antibody" refers to an antibody in which the framework of the antibody is a human antibody and a part of the CDR region is modified to include only the portion of the CDRs of the species from which the original antibody molecule is derived that is essential for specific binding to the antigen. For example, the CDR portions other than the portion of the CDRs of a monkey- or mouse-derived antibody that is essential for specific binding to the antigen, and the light chain and heavy chain frameworks, are replaced with those of a human antibody.

In addition, according to the present invention, the antibody of the present invention can be produced in various forms of antibodies. The antibody of the present invention can be produced as a multifunctional fusion antibody by fusing Fabs having different functions, for example, a CD40+CD40L fusion antibody, and a whole-form antibody can also be provided by recombining the light chain and heavy chain variable regions obtained from a Fab antibody with a constant region of human origin.

In addition, the anti-CD40 antibody or antigen-binding fragment thereof of the present invention may be a monoclonal antibody. The term "monoclonal antibody" or "single-clone antibody" refers to an antibody molecule of a single molecular composition obtained from a substantially identical antibody population, and a monoclonal antibody exhibits a single binding specificity and affinity for a particular epitope.

A monoclonal antibody can be prepared by various methods known in the art, which are fundamentally based on fusing myeloma cells with splenocytes derived from an immunized mammal.

The antibody according to the present application may, depending on the specific purpose, additionally be provided in conjugation with a functional substance selected from the group consisting of a therapeutic agent, a prodrug, a peptide, a protein, an enzyme, a virus, a lipid, a biological modifier, a drug, and PEG (polyethylene glycol). In addition, it may be prepared using various methods depending on the type of substance to be conjugated.

As the therapeutic agent, prodrug, peptide, protein, enzyme, virus, lipid, biological modifier, and drug, any of those commonly used in the art may be used, as long as the intended effect can be achieved.

Fragments of the antibody can be obtained by treatment with pepsin or papain. An F(ab')2 fragment can be obtained by treating an intact antibody with pepsin, and subsequent treatment thereof with a thiol reducing agent yields a Fab fragment comprising the light chain and a portion of the heavy chain. A Fab fragment can also be obtained by treating an intact antibody with papain. For example, an antibody fragment that specifically recognizes CD40, such as F(ab')2 or Fab, can be prepared by treating an antibody produced in the hybridoma herein with pepsin or papain.

An Fv fragment is an antibody fragment composed only of the variable regions of the heavy chain and the light chain, and the two variable regions can be linked by a noncovalent or covalent bond, such as a chemical crosslinker or an intermolecular disulfide bond (Inbar et al. (1972) PNAS 69:2659-2662). For example, an antibody that specifically recognizes CD40 can be prepared by enzymatically treating an antibody produced in the hybridoma herein to isolate only the variable regions of the heavy chain and the light chain, or by using recombinant DNA technology.

An SCA fragment can be prepared enzymatically or by genetic engineering, and is an antibody fragment in which the variable region of the light chain and the variable region of the heavy chain are linked by a linker, such as a polypeptide. For methods of preparing scFv, reference may be made, for example, to those described in U.S. Patent No. 4,936,778 or U.S. Patent No. 5,892,019. An antibody that specifically recognizes CD40 can be prepared by enzymatically treating an antibody produced in the hybridoma herein, or by recombinant DNA technology, for example, by constructing a vector comprising a nucleic acid sequence encoding the heavy chain and/or light chain variable region of the antibody and expressing the vector in a suitable cell.

As used herein, the term "binding" or "specific binding" refers to the affinity of the antibody or antibody composition herein for an antigen. In antigen-antibody binding, "specific binding" can typically be distinguished from non-specific background binding when the dissociation constant (Kd) is less than 1×10⁻⁵ M, less than 1×10⁻⁶ M, or less than 1×10⁻⁷ M. Specific binding can be detected by methods known in the art, for example, ELISA, SPR (Surface Plasmon Resonance), immunoprecipitation, and coprecipitation, and includes appropriate controls capable of distinguishing non-specific binding from specific binding.

In the present invention, the anti-CD40 antibody may be selected from the group consisting of a multimeric antibody, a heterodimeric antibody, a half antibody, a tetravalent antibody, a bispecific antibody, and a single-chain antibody, but is not limited thereto.

The antibody herein, which comprises an intact antibody or a fragment thereof as described above, may exist as a multimer, such as a dimer, trimer, tetramer, or pentamer, comprising at least a part of the antigen-binding capacity of the monomer. Such multimers also include homomultimers or heteromultimers. Since an antibody multimer comprises multiple antigen-binding sites, it has superior antigen-binding capacity compared to a monomer. Antibody multimers are also useful for the preparation of multifunctional (bifunctional, trifunctional, tetrafunctional) antibodies.

As used herein, the term "multifunctional" refers to an antibody or antibody composition having two or more activities or functions (e.g., antigen-binding capacity, enzyme activity, ligand or receptor binding capacity). For example, the antibody herein can be linked to a polypeptide having enzyme activity, such as luciferase, acetyltransferase, or galactosidase. A multifunctional antibody also includes antibodies in a multivalent or multispecific (bispecific, trispecific, etc.) form.

As used herein, the term "multispecific" refers to comprising a variable region capable of binding to two or more different epitopes. The two or more epitopes may be present on one antigen or may be present on different antigens.

In the present invention, the antigen-binding fragment may be selected from the group consisting of Fab, F(ab)2, Fab', F(ab)2, F(ab')3, Fd, Fv, and a domain antibody, but is not limited thereto.

Another embodiment of the present invention provides a nucleic acid encoding the anti-CD40 antibody or antigen-binding fragment thereof.

The nucleic acid includes, for example, DNA, cDNA, RNA, or recombinant or synthetic DNA or RNA. In one embodiment, the nucleic acid molecule is cDNA. The nucleic acid may also be the corresponding genomic DNA or a fragment thereof. Nucleic acid sequences encoding the antibody, or a portion or fragment thereof, according to the present application may differ due to the redundancy of nucleic acid sequences encoding amino acids, and such sequences are also included herein.

Another embodiment of the present invention provides a vector comprising the nucleic acid.

Another embodiment of the present invention provides a host cell comprising the nucleic acid.

Another embodiment of the present invention provides a method for producing an antibody, comprising culturing the host cell to express the antibody.

Vectors that can be used herein include, for example, phage, plasmid, and replicable or non-replicable viral or retroviral vectors. The nucleic acid molecule according to the present application can be introduced into various known vectors. For example, vectors for prokaryotic cells include pUC-series vectors, pBluescript (Stratagene), pET-series vectors (Novagen), and pCRTOPO (Invitrogen) vectors; and vectors for eukaryotic cells include, but are not limited to, pREP (Invitrogen), pcDNA3 (Invitrogen), pCEP4 (Invitrogen), pMCI neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems), pTriEx-Hygro (Novagen), and pCINeo (Promega) vectors.

The vector according to the present application can be introduced into various known prokaryotic or eukaryotic cells by known transformation or transfection methods. Upon introduction into a cell, it may be inserted into the genome of the host cell or may exist in an extrachromosomal form.

Prokaryotic cells that can be used include cells belonging to the *Escherichia*, *Bacillus*, *Streptomyces*, and *Salmonella* lineages; and eukaryotic cells include, but are not limited to, mammalian cells, for example, HeLa, HEK293, H9, Jurkat, mouse NIH3T3, C127, Cos1, Cos7, and CV1, mouse C2C12, BHK, and CHO cells; fungal cells, such as *Saccharomyces cerevisiae* or *Pichia pastoris*; and insect cells, such as *Drosophila* S2 and *Spodoptera* Sf9.

The antibody herein can be produced according to known methods using recombinant techniques. In the case of recombinant methods, a nucleic acid sequence encoding the heavy chain of the antibody according to the present application and a nucleic acid encoding the light chain of the antibody are cloned into one or two expression vectors, and then transfected into a eukaryotic host cell to express the antibody, after which the antibody can be obtained from the host cell or the medium. Recombinant methods, including the construction of such vectors, the expression of the protein in cells from the constructed vectors, and the isolation of the protein, are known in the art, and reference may be made, for example, to those described in Kaufman, RJ, Mol (2000) Biotechnol 16:151-160. A vector encoding the antibody herein can be expressed in a suitable host cell, for example, a CHO cell, NS0 cell, SP2/0 cell, HEK293 cell, COS cell, yeast, or E. coli, and the antibody can be obtained from a cell lysate or the medium.

A nucleic acid sequence encoding all or a fragment of the antibody herein can be isolated from the hybridoma cells disclosed herein according to conventional methods, after which its sequence can be analyzed. The isolated nucleic acid sequence is then cloned into a suitable expression vector as described above and transfected into HEK293 cells, CHO cells, or NS0 cells that do not produce antibodies, to produce a recombinant antibody in the host cells. A nucleic acid encoding the antibody or a fragment thereof herein is introduced into an expression vector comprising a promoter, a translation initiation site, a 3' untranslated region, a polyadenylation signal, and a transcription termination signal. The light chain and the heavy chain may be introduced into one vector or into separate vectors.

Another embodiment of the present invention provides an antibody-drug conjugate comprising the anti-CD40 antibody or antigen-binding fragment thereof as an active ingredient.

As used herein, the term "antibody-drug conjugate (ADC)" refers to a form in which a drug and an antibody are chemically linked without reducing the biological activity of the antibody and the drug. In the present invention, the antibody-drug conjugate refers to a form in which a drug is bound to an amino acid residue at the N-terminus of the heavy chain and/or light chain of the antibody, specifically, a form in which a drug is bound to the N-terminal α-amine group of the heavy chain and/or light chain of the antibody.

As used herein, the term "drug" may refer to any substance having a particular biological activity on a cell, and is a concept encompassing compounds, DNA, RNA, peptides, and the like. It may be in a form comprising a reactive group capable of reacting with and crosslinking to the α-amine group, and also includes a form in which a linker comprising a reactive group capable of reacting with and crosslinking to the α-amine group is connected. In this case, the drug may be bound site-specifically to the N-terminal amino acid residue of the antibody by the linker, but is not limited thereto. The linker refers to a chemical moiety comprising an atomic chain that covalently bonds the drug to the antibody. The linker is prepared in a form linked to the drug, and has, at its end, a reactive group capable of being linked to the antibody.

In the present invention, the drug includes all substances capable of causing a cell to undergo cell death, cell proliferation, immune activation, or immune suppression, including the activation of particular signaling or the inhibition of particular signaling, and the drug may in particular be a cytotoxic drug or an immunosuppressant.

In the present invention, the cytotoxic drug includes chemotherapeutic agents capable of functioning as a microtubulin structure formation inhibitor, a mitosis (meiosis) inhibitor, an RNA polymerase inhibitor, a topoisomerase inhibitor, a DNA intercalator, a DNA alkylator, a ribosome inhibitor, and the like, protein toxins capable of functioning enzymatically, and radioisotopes. Examples thereof may include maytansinoid, auristatin, dolastatin, tubulysin, calicheamicin, pyrrolobenzodiazepines, doxorubicin, duocarmycin, carboplatin (paraplatin), cisplatin, cyclophosphamide, ifosfamide, nidran, nitrogen mustard (mechlorethamine HCl), bleomycin, mitomycin C, cytarabine, fluorouracil, gemcitabine, trimetrexate, methotrexate, etoposide, vinblastine, vinorelbine, alimta, altretamine, procarbazine, taxol, taxotere, topotecan, irinotecan, trichothecene, CC1065, alpha-amanitin, other enediyne antibiotics, exotoxins, and plant toxins. In the case of a compound, stereoisomers and derivatives thereof are also included. In addition, the auristatin may be monomethyl auristatin E or monomethyl auristatin F, but is not limited thereto.

In the present invention, the drug may be selected from the group consisting of an anticancer agent, an aptamer, an antisense oligonucleotide, a small interfering RNA (siRNA), and a microRNA, but is not limited thereto.

Another embodiment of the present invention relates to a pharmaceutical composition for preventing or treating an autoimmune disease, comprising the antibody or antigen-binding fragment thereof as an active ingredient.

In the present invention, the autoimmune disease may be a disease selected from the group consisting of type 1 diabetes, new-onset type 1 diabetes, lymphoma, chronic lymphocytic leukemia, alopecia areata, ankylosing spondylitis, Sjögren's syndrome, Evans syndrome, lupus, autoimmune nephritis 2, antiphospholipid syndrome, autoimmune Addison's disease, autoimmune disease of the adrenal gland, autoimmune hemolytic anemia, autoimmune hepatitis, autoimmune oophoritis and orchitis, autoimmune thrombocytopenia, Behçet's disease, bullous pemphigoid, cardiomyopathy, celiac sprue-dermatitis, chronic fatigue immune dysfunction syndrome, chronic inflammatory demyelinating polyneuropathy, Churg-Strauss syndrome, cicatricial pemphigoid, CREST syndrome, cold agglutinin disease, Crohn's disease, discoid lupus, essential mixed cryoglobulinemia, fibromyalgia-fibromyositis, glomerulonephritis, focal segmental glomerulosclerosis, Graves' disease, Guillain-Barré syndrome, Hashimoto's thyroiditis, idiopathic pulmonary fibrosis, idiopathic thrombocytopenic purpura, inflammatory bowel disease, IgA neuropathy, neuromyelitis optica, juvenile arthritis, lichen planus, lupus erythematosus, Ménière's disease, mixed connective tissue disease, amyotrophic lateral sclerosis, multiple sclerosis, hidradenitis suppurativa, myasthenia gravis, pemphigus vulgaris, pernicious anemia, microscopic polyangiitis, polyarteritis nodosa, polychondritis, autoimmune polyglandular syndrome, polymyalgia rheumatica, polymyositis and dermatomyositis, primary agammaglobulinemia, primary biliary cirrhosis, psoriasis, psoriatic arthritis, hepatic fibrosis, Raynaud's phenomenon, Reiter's syndrome, rheumatoid arthritis, sarcoidosis, scleroderma, stiff-person syndrome, systemic lupus erythematosus, lupus erythematosus, Takayasu's arteritis, temporal arteritis, giant cell arteritis, ulcerative colitis, uveitis, vitiligo, graft-versus-host disease, opsoclonus-myoclonus syndrome, Addison's disease, and Wegener's granulomatosis, but is not limited thereto.

Another embodiment of the present invention provides a pharmaceutical composition for preventing or treating a chronic inflammatory disease, comprising the antibody or antigen-binding fragment thereof as an active ingredient.

As used herein, the term "prevention" may refer to any action that inhibits or delays the onset of a disease by administration of the composition. In addition, "treatment" may refer to any action that ameliorates or beneficially alters the symptoms of a disease by administration of the composition.

In the present invention, the chronic inflammatory disease may be a disease selected from the group consisting of inflammatory bowel disease, Crohn's disease, ulcerative colitis, pancreatitis, chronic hepatitis, esophagitis, gastritis, colitis, pneumonia, bronchitis, pharyngitis, myocardial infarction, heart failure, Alzheimer's disease, psoriatic arthritis, rheumatoid arthritis, multiple sclerosis, systemic lupus erythematosus, asthma, renal failure, psoriasis, anemia, diabetes, and fibrosis, but is not limited thereto.

Another embodiment of the present invention provides a composition for suppressing immune rejection upon transplantation, comprising the antibody or antigen-binding fragment thereof as an active ingredient.

As used herein, the term "immune rejection upon transplantation" refers to a phenomenon of destruction caused by a specific immune response against foreign cells or tissues inoculated or transplanted from a donor into a recipient. A graft transplanted from a donor having different transplantation antigens cannot engraft by the immunological mechanisms of the recipient, and such rejection is caused by cellular immunity and humoral immunity. In the transplantation of skin or solid tumors, the main agent of rejection is cellular immunity; that is, the transfer of serum from an animal that has rejected a transplanted skin graft does not promote rejection of the same graft in the recipient animal, but the transfer of lymphoid cells shortens the engraftment period of the graft. In addition, an animal whose thymus was excised in the neonatal period does not exhibit skin rejection, whereas in renal rejection, humoral antibodies also act as a main agent of rejection; in particular, when the recipient already has antibodies against the donor lymphocytes, microthrombi are formed in the glomeruli, causing hyperacute rejection. Acute rejection occurs between 7 and 21 days after transplantation, in which case both cellular immunity and humoral antibodies are involved. In addition, chronic rejection occurs from several months to several years after transplantation, which is known to be caused by the deposition of immunoglobulins and C3 in the glomerular basement membrane.

In the present invention, the transplantation may be transplantation of at least one selected from the group consisting of allogeneic cells, xenogeneic cells, allogeneic tissues, xenogeneic tissues, allogeneic organs, and xenogeneic organs.

In the present invention, the transplantation may be the suppression of immune rejection upon transplantation of skin, blood, cornea, liver, lung, intestine, pancreas, heart, kidney, bone marrow, stem cells, or progenitor cells.

The composition of the present invention is formulated together with a pharmaceutically acceptable carrier and, optionally, an excipient or stabilizer.

As used herein, the term "pharmaceutically acceptable carrier" refers to a carrier or diluent that does not irritate the organism and does not impair the biological activity and properties of the administered compound. In a composition formulated as a liquid solution, acceptable pharmaceutical carriers include those that are sterile and biocompatible, such as saline, sterile water, Ringer's solution, buffered saline, albumin injection solution, dextrose solution, maltodextrin solution, glycerol, and ethanol, and a mixture of one or more of these components may be used; and, if necessary, other conventional additives, such as antioxidants, buffers, and bacteriostatic agents, may be added. In addition, by additionally adding a diluent, a dispersant, a surfactant, a binder, and a lubricant, it can be formulated into an injectable formulation, such as an aqueous solution, suspension, or emulsion, a pill, a capsule, a granule, or a tablet.

The composition may be in various oral or parenteral dosage forms. When formulated, it is prepared using a diluent or excipient, such as a filler, a bulking agent, a binder, a wetting agent, a disintegrant, or a surfactant, that is commonly used. Solid preparations for oral administration include tablets, pills, powders, granules, capsules, and the like, and such solid preparations are prepared by mixing at least one excipient, for example, starch, calcium carbonate, sucrose or lactose, or gelatin, with one or more compounds. In addition to simple excipients, lubricants, such as magnesium stearate and talc, are also used. Liquid preparations for oral administration include suspensions, solutions for internal use, emulsions, and syrups, and may include, in addition to water and liquid paraffin, which are commonly used simple diluents, various excipients, for example, wetting agents, sweeteners, fragrances, and preservatives. Preparations for parenteral administration include sterile aqueous solutions, non-aqueous solvents, suspensions, emulsions, lyophilized preparations, and suppositories. As the non-aqueous solvent and suspending solvent, propylene glycol, polyethylene glycol, vegetable oils such as olive oil, and injectable esters such as ethyl oleate may be used. As the base of the suppository, witepsol, macrogol, tween 61, cacao butter, laurin butter, glycerogelatin, and the like may be used.

The composition according to the present invention can be administered by various routes known in the art, and it is obvious to those skilled in the art that the mode and route of administration may differ depending on the desired effect. The composition of the present invention can be administered orally or parenterally, and is preferably administered parenterally. When administered parenterally, the composition of the present invention can be administered by intravenous injection, subcutaneous injection, intramuscular injection, intraperitoneal injection, and the like. The route of administration of the composition of the present invention is preferably determined depending on the type of disease to which it is applied.

The effective dose and administration period of the composition according to the present invention may vary depending on the desired therapeutic effect, taking into consideration the particular patient, the type of antibody included in the composition, and the mode of administration, and must not cause toxicity to the patient. The actual dose for each patient should be selected taking into consideration various factors, such as the activity of the composition used, the route of administration, the time of administration, the rate of secretion, other drugs used together, sex, age, body weight, general health condition, and underlying disease. In one embodiment, the antibody herein may be administered for the treatment or prevention of a disease in an amount of about 1 to 100 mg/kg body weight, for example, about 10, 20, 30, 40, or 50 mg/kg body weight, but, in some cases, may be administered in an amount of as much as about 100 mg/kg.

The administration interval of the composition according to the present invention may be administered at appropriate intervals on a daily, weekly, or monthly basis, taking into consideration the half-life of the administered antibody.

In addition, the composition according to the present invention may be formulated, regardless of the route of administration, in a suitable pharmaceutically acceptable dosage form, for example, a hydrated form, such as an aqueous solution, or a lyophilized form.

Another embodiment of the present invention provides a composition for reducing or preventing the formation of an anti-drug antibody (ADA) against a therapeutic agent, comprising the antibody or antigen-binding fragment thereof according to the present invention as an active ingredient.

As used herein, the term "anti-drug antibody" is an antibody that may be directed against any region of a drug, such as, for example, a variable domain, a constant domain, or a sugar structure of the drug. Such anti-drug antibodies may arise from an immunogenic response of a patient during antibody therapy (see Pan, Y., et al., FASEB J. 9 (1995) 43-49). Most anti-drug antibodies bind to one or more complementarity determining regions of the drug. The affinity of an anti-drug antibody for the antigen of the drug is generally lower than the affinity of the drug for its target antigen.

As used herein, the term "drug" refers to a therapeutic protein or a therapeutically effective portion thereof that can be administered to a subject for the treatment of a disease.

In the present invention, the therapeutic agent may preferably be an antibody therapeutic agent, a cell therapeutic agent, a gene therapeutic agent, a biologic, or a peptide therapeutic agent, but is not limited thereto.

Another embodiment of the present invention provides a chimeric antigen receptor (CAR) comprising the anti-CD40 antibody or antigen-binding fragment thereof according to the present invention as an active ingredient, and a T cell (CAR-T) or NK cell (CAR-NK) engineered to express the same.

As used herein, the term "chimeric antigen receptor" refers to a fusion protein comprising an extracellular domain that binds to an antigen, a transmembrane domain derived from a polypeptide different from the extracellular domain, and at least one intracellular domain. The chimeric antigen receptor is also called a chimeric receptor, a T-body, or a chimeric immune receptor (CIR). The extracellular domain that binds to an antigen refers to any oligo- or polypeptide capable of binding to a certain antigen, and the intracellular domain refers to any oligo- or polypeptide known to function as a domain that transmits a signal that brings about the activation or inhibition of a biological process within the cell.

Another embodiment of the present invention provides a method for preventing or treating an autoimmune disease, a chronic inflammatory disease, or the like, comprising administering, to a subject in need of treatment, a composition comprising the anti-CD40 antibody or antigen-binding fragment thereof according to the present invention as an active ingredient.

In the present invention, the subject is an animal that is the target of treatment, observation, or experiment, and preferably includes mammals, birds, and the like, including cattle, pigs, sheep, chickens, dogs, and humans; and a subject whose disease is treated by administration of the composition of the present invention is included without limitation.

### [ADVANTAGEOUS EFFECTS OF INVENTION]

The novel anti-CD40 antibody of the present invention blocks CD40-CD40L signaling to exhibit an excellent antagonistic effect, and thus can be widely utilized as an agent for preventing or treating various diseases, such as autoimmune diseases and chronic inflammatory diseases, and as an agent for suppressing immune rejection upon transplantation.

### [BRIEF DESCRIPTION OF DRAWINGS]

FIG. 1 shows the CDR sequences of the 8G1-35-31 antibody according to one embodiment of the present invention.
FIG. 2 shows the results of a CD40 antigen-specific binding assay according to one embodiment of the present invention.
FIG. 3 shows the results of confirming binding to a CD40-expressing cell line, the Raji B cell line, according to one embodiment of the present invention.
FIG. 4 shows the results of confirming the concentration-dependent binding ability of the 8G1-35-31 antibody to the human CD40 antigen according to one embodiment of the present invention.
FIG. 5 shows the CD40-CD40L binding blocking effect of the 8G1-35-31 antibody according to one embodiment of the present invention.
FIG. 6 shows the inhibitory effect on T-dependent B-cell activation by treatment with the 8G1-35-31 antibody according to one embodiment of the present invention.
FIG. 7 shows the inhibitory effect on T-dependent B-cell proliferation by treatment with the 8G1-35-31 antibody according to one embodiment of the present invention.
FIG. 8 shows the results of a non-agonist property assay of the 8G1-35-31 antibody according to one embodiment of the present invention.
FIG. 9 shows the results of investigating the binding ability of the 8G1-35-31 antibody to a linear CD40 antigen according to one embodiment of the present invention.
FIG. 10 shows the results of investigating interspecies cross-binding ability according to one embodiment of the present invention.
FIG. 11 shows the KLH-specific antibody secretion inhibitory effect of the 8G1-35-31 antibody according to one embodiment of the present invention.
FIG. 12 shows the KLH-specific Th1-cell activation inhibitory effect of the 8G1-35-31 antibody according to one embodiment of the present invention.

### [DESCRIPTION OF EMBODIMENTS]

Hereinafter, the present invention will be described in more detail through the following Examples. However, the following Examples are only for illustrating the present invention, and the scope of the present invention should not be construed as being limited to these Examples. The following Examples are provided to more completely explain the present invention to those of ordinary skill in the art.

### Example 1: Preparation of a Hybridoma Producing an Anti-CD40 Antibody

The present inventors prepared, selected, and analyzed a novel anti-CD40 antibody that targets CD40 to block CD40-CD154 (CD40L) signaling, as follows.

To develop a novel anti-CD40 antibody, human CD40 antigen (100 µg/mouse) or a CD40-expressing CHO cell line in a number of 1×10⁷ was injected three times at three-week intervals into the intraperitoneal cavity (IP) of each 6-week-old Balb/c female mouse, and a blood sample was taken from a vein to separate the serum. The separated serum diluted in CD40-expressing CHO cells was added, allowed to react at 4°C for 30 minutes, and then 3 ml of PBS was added, followed by centrifugation at 1500 rpm for 3 minutes to wash off the unbound antibody. To confirm the bound antibody, the secondary antibody goat anti-Mouse Ig-FITC (Invitrogen) was diluted 200-fold and added, allowed to react at 4°C for 15 minutes, washed with 3 ml of PBS in the same manner as above, and then measured with a flow cytometer to confirm the titer against the CD40-expressing CHO cell line. As a result of confirming the titer against the CD40-expressing CHO cell line by flow cytometry, it was confirmed that the serum immunized with the CD40-expressing CHO cell line showed high positivity against the CD40-expressing CHO cell line.

The spleen of the immunized mouse as above was excised to obtain a single-cell suspension, which was washed about twice with RPMI (GIBCO), and then 0.4% trypan blue (Sigma) was mixed at 1:1 (v/v), and the number of cells was counted by the trypan blue staining method, in which unstained cells were measured under a microscope. As the cell-fusion partner cell, the X63 mouse myeloma cell line (ATCC CRL-1580) was used, and after washing in the same manner as the splenocytes above, the number of cells was counted.

The myeloma cells and the splenocytes were mixed at a ratio of 1:5, and after centrifugation, the supernatant was removed. 1 ml of 50% PEG (polyethylene glycol) 1500, preheated to 37°C in advance, was slowly added over 1 minute. After standing for about 1 minute, RPMI medium was slowly added to dilute it stepwise. After centrifuging this, it was suspended in RPMI (20% FBS) containing 1× HAT (hypoxanthine-aminopterin-thymidine), dispensed at 150 µl/well into a 96-well plate, and then cultured in a 37°C, 5% CO₂ incubator. After the fusion, HAT feeding was carried out for a certain period, and when wells forming colonies were observed, 150 µl of HT medium was added, followed by additional culture in a 37°C, 5% CO₂ incubator for 2-7 days, after which the culture medium was obtained. The culture medium was subjected to fluorescence staining using the CD40-expressing CHO cell line and analyzed with a flow cytometer.

To the CD40-expressing CHO cell line, 100 µl each of the hybridoma culture supernatant was added, allowed to react at 4°C for 30 minutes, and then 3 ml of PBS was added, followed by centrifugation at 1500 rpm for 3 minutes to wash off the unbound antibody. To confirm the bound antibody, the secondary antibody goat anti-Mouse Ig-FITC (Invitrogen) was diluted 200-fold and added, allowed to react at 4°C for 15 minutes, washed with 3 ml of PBS in the same manner as above, and then measured with a flow cytometer.

In the above method, monoclonal antibodies that were negative for CHO cells but positive for the CD40-expressing CHO cell line or the B cell line (Raji) were selected, and finally, limiting dilution was repeated three times to secure single-colony 8G1-35-31 hybridoma cells.

The hybridoma was selected as the optimal hybridoma and designated 8G1-35-31.

The hybridoma 8G1-35-31 was deposited with the Korean Cell Line Research Foundation on June 1, 2023, and was assigned the following accession number: KCLRF-BP-00523.

### Example 2: Confirmation of the Sequence of the Anti-CD40 Antibody

In order to analyze the structure of the antibody produced according to the present invention, the present inventors cloned nucleic acids encoding the heavy chain and light chain fragments from the hybridoma producing the anti-CD40 antibody.

The cloning and sequence determination were carried out as follows.

For the cloning of the genes, RNA was extracted from the 8G1-35-31 hybridoma cells obtained in Example 1 using an RNA miniprep kit (Qiagen) according to the manufacturer's method, after which PCR was performed to synthesize cDNA.

The PCR conditions and cloning method for the synthesis of the heavy chain cDNA and the light chain cDNA are as follows:

The CD40 antigen-specific antibody, the RM8G-1 antibody gene, was cloned using a Mouse Ig-Primer Set (Millipore, Cat #: 69831). PCR was performed using the Mouse Ig-Primer Set from the RNA isolated from the RM8G-1 hybridoma, the product was inserted into a pGem-T vector (Promega, Cat #: A3600), the DNA nucleotide sequence was confirmed through sequencing, and the mouse antibody gene was confirmed through the IMGT site (www.imgt.org).

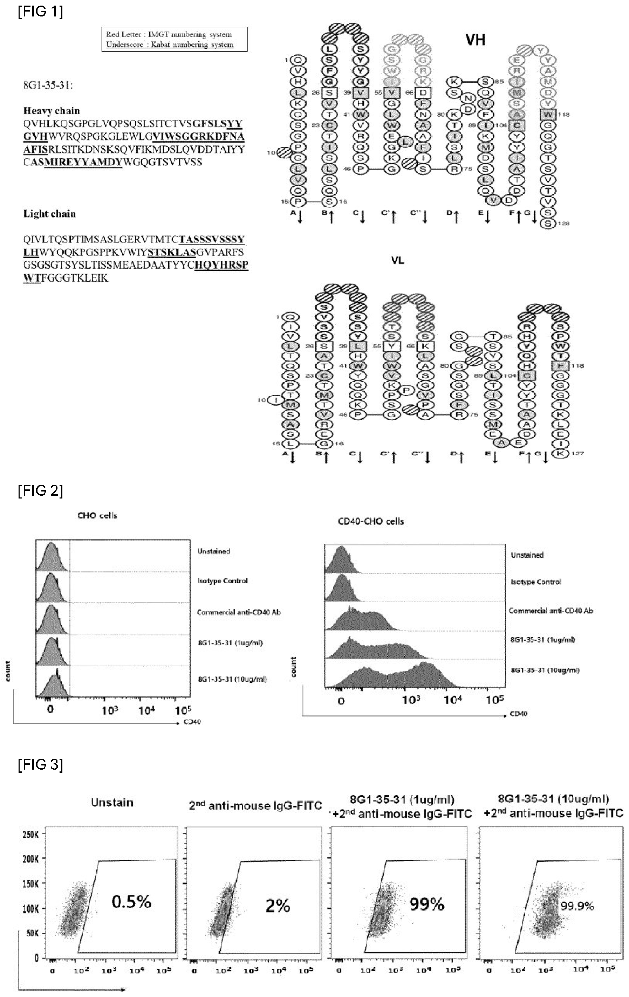

The sequences of the heavy chain variable region HCDR1, HCDR2, and HCDR3, and the sequences of the light chain variable region LCDR1, LCDR2, and LCDR3, are shown in Table 1 below. In addition, the full sequences of the heavy chain variable region and the light chain variable region are as set forth in SEQ ID NOs: 13 and 14, respectively (FIG. 1).

**[Table 1]**

| | IMGT format | Kabat format |
|---|---|---|
| HCDR1 | GFSLSYYG (SEQ ID NO: 1) | YYGVH (SEQ ID NO: 7) |
| HCDR2 | IWSGGRK (SEQ ID NO: 2) | VIWSGGRKDFNAAFIS (SEQ ID NO: 8) |
| HCDR3 | ASMIREYYAMDY (SEQ ID NO: 3) | MIREYYAMDY (SEQ ID NO: 9) |
| LCDR1 | SSVSSSY (SEQ ID NO: 4) | TASSSVSSSYLH (SEQ ID NO: 10) |
| LCDR2 | STS (SEQ ID NO: 5) | STSKLAS (SEQ ID NO: 11) |
| LCDR3 | HQYHRSPWT (SEQ ID NO: 6) | HQYHRSPWT (SEQ ID NO: 12) |

### Example 3: Confirmation of the Antagonistic Effect of the Anti-CD40 Antibody

### 3-1: CD40 Antigen-Specific Binding Assay

To investigate whether the 8G1-35-31 antibody specifically binds to the human CD40 antigen (hCD40), flow cytometry was performed using Chinese Hamster Ovary cells (CHO) and hCD40 antigen-expressing CD40-CHO cells. CHO cells and 2×10⁵ CD40-CHO cells were each treated with an isotype control (mouse IgG-FITC, Biolegend), a commercial anti-CD40 antibody (5C3, BD), 8G1-35-31-FITC (1 µg/ml), and 8G1-35-31-FITC (10 µg/ml), and allowed to react on ice for 30 minutes. After washing with FACS buffer (5% BSA, 0.01% Sodium Azide, PBS, pH 7.4), analysis was performed using a BD FACSCANTO II. As a result, CD40 staining did not occur in CHO cells that do not express CD40, whereas the CD40 antigen was detected in the samples in which the commercial antibody and 8G1-35-31 were applied to CD40-CHO. This confirmed that 8G1-35-31 specifically binds to the human CD40 antigen (FIG. 2).

### 3-2: Confirmation of Binding to a CD40-Expressing Human B Cell Line (Raji B Cells)

To confirm whether the 8G1-35-31 antibody binds to CD40 antigen-expressing human B cells, flow cytometry was performed using Raji B (ATCC) cells. 2×10⁵ Raji B cells were each treated with 8G1-35-31 at concentrations of 1 µg/ml and 10 µg/ml and allowed to react on ice for 30 minutes. Thereafter, after washing twice with FACS buffer, anti-mouse IgG-FITC (Sigma) was applied at a concentration of 1 µg/ml and allowed to react on ice for 40 minutes. After washing twice with FACS buffer, analysis was performed using a BD FACSCANTO II. As a result, it was confirmed that the 8G1-35-31 antibody binds to CD40-expressing Raji B cells (FIG. 3).

### 3-3: Confirmation of Concentration-Dependent Binding Ability

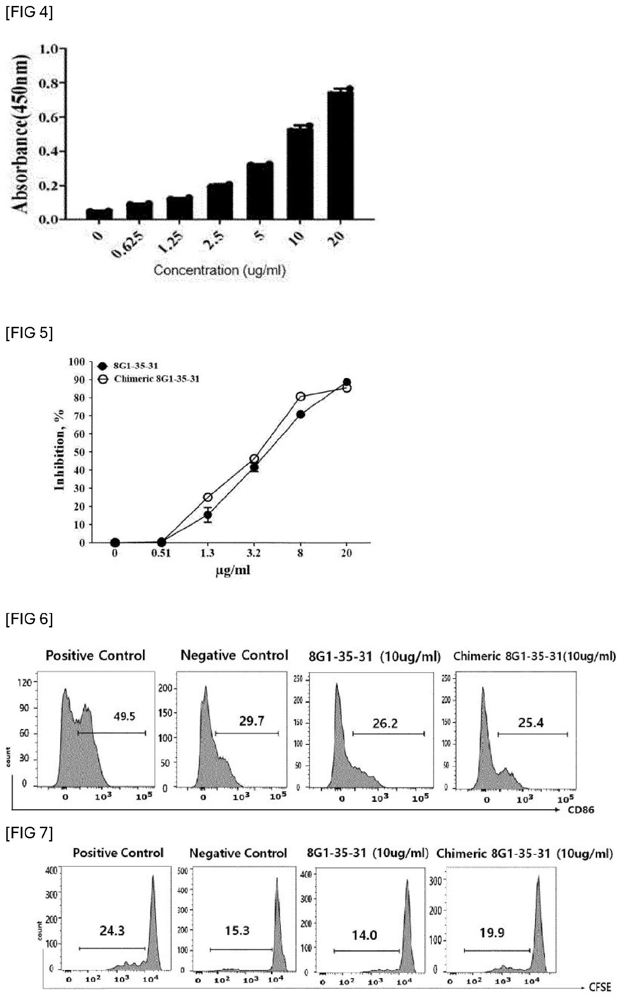

To confirm the concentration-dependent binding ability of the 8G1-35-31 antibody, an ELISA (Enzyme-Linked Immunosorbent Assay) was performed. CD40-his (1 µg/mL, Sino Biological, Cat#. 10774-H08H) diluted in ELISA Phosphate Coating Buffer (Thermo Scientific, Cat#. CB07100) was reacted in a 96-well ELISA plate (Thermo Scientific, Cat#. 439454) at 37°C for 1 hour. The test sample 8G1-35-31 to be used in the experiment was diluted (0-20 µg/ml), and 100 µL each was added and allowed to react at 37°C for 1 hour. After washing three times with 200 µl of PBST (10 mM sodium phosphate, 0.15 M NaCl, 0.05% Tween^{™} 20 buffer at pH 7.5), 50 µl of a diluted IgG Fc Goat anti-Human IgG Fc Cross-Adsorbed Secondary Antibody-HRP (1:5000) solution was added and allowed to react at 37°C for 30 minutes. After washing three times with 200 µl of PBST, 50 µL each of TMB substrate solution was dispensed, light was blocked with foil, and the reaction was carried out at room temperature for 5 minutes. Thereafter, 50 µl of stop solution (Stop Solution, Thermo Scientific, Cat# N600) was further added to stop the reaction, and the absorbance was measured at a wavelength of 450 nm using a microplate ELISA reader (Varioskan Lux microplate reader, Thermo Fisher Scientific, Cat#. VLBL0TD2). As a result, it was confirmed that the 8G1-35-31 antibody exhibits a concentration-dependent binding pattern in which the binding ability increases according to the amount of the CD40 antigen (FIG. 4).

### 3-4: CD40-CD40L Binding Blocking Effect Using Reporter Cells

Using HEK-Blue human CD40L-SEAP Reporter cells (hereinafter referred to as HEK-Blue CD40L, InvivoGen, Cat#. hkb-cd40), the CD40L-CD40 binding blocking effect was quantified at the cellular level. That is, the HEK-Blue CD40L cell suspension was dispensed into a 96-well plate to a concentration of 2.5×10⁵/ml, and a recombinant CD40L solution (InvivoGen, Cat#. rcyec-hcd40) was added at a concentration of mL. A human-mouse antibody chimeric 8G1-35-31 antibody was produced by combining the nucleotide sequence of the variable region of the mouse 8G1-35-31 antibody with the nucleotide sequence of the human Fc region. The quantified 8G1-35-31 antibody and chimeric 8G1-35-31 antibody were added at various concentrations to the binding blocking experimental model using the reporter cells (three replicates per experimental group) and cultured in an incubator at 37°C, humidified, 5% CO₂ conditions for 20 hours. Thereafter, 20 µl of the cultured supernatant was taken and transferred to a 96-well plate, and 180 µl of QUANTI-Blue^{™} Solution (InvivoGen, Cat#. rep-qbs) was added and allowed to react at room temperature for 30 minutes. The binding blocking ability was investigated based on the value obtained by measuring at 620 nm with a microplate reader. The equation for calculating the binding blocking ability is shown in the following Mathematical Formula 1. [Mathematical Formula 1] 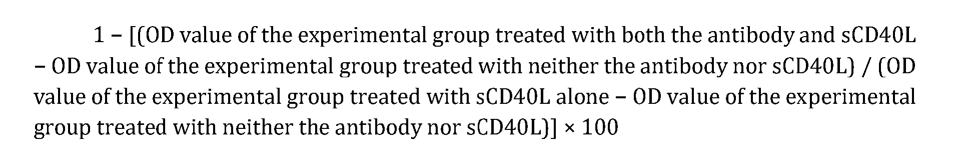 1 - [(OD value of the experimental group treated with both the antibody and sCD40L - OD value of the experimental group treated with neither the antibody nor sCD40L) / (OD value of the experimental group treated with sCD40L alone - OD value of the experimental group treated with neither the antibody nor sCD40L)] × 100

As a result, it was confirmed that the 8G1-35-31 antibody can effectively block CD40-CD40L signaling in a concentration-dependent manner in a binding blocking experiment using CD40-CD40L binding reporter cells (FIG. 5).

### 3-5: T-Cell-Dependent B-Cell Activation and Proliferation Inhibitory Effect Using PBMCs

In a T-cell-dependent B-cell activation model in human PBMCs, the degree of inhibition of the expression of CD86, a B-cell activation factor, by the 8G1-35-31 drug was quantified and confirmed. That is, an activating anti-CD3 antibody (OKT3) and an activating anti-CD28 antibody (CD28.6) were dissolved in phosphate-buffered saline (PBS, pH 7.4), added to a 96-well U-bottom plate at a concentration of 5 µg/ml, and coated at 4°C for 18 hours. The next day, human PBMCs stained with CFSE (2.5 µM) were placed in the anti-CD3/anti-CD28 antibody-coated 96-well plate at a concentration of 2.5×10⁶/ml, and the 8G1-35-31 antibody at each concentration (10 µg/ml, 100 µg/ml) was added, followed by culture in a 37°C, humidified, 5% CO₂ incubator for 4 or 5 days. For the positive control, only the anti-CD3/anti-CD28 antibody-coated plate was used, and 8G1-35-31 was not added; and for the negative control, neither the anti-CD3/anti-CD28 antibody nor the 8G1-35-31 antibody was added. After 4-5 days, the cultured cells were transferred to a FACS tube (USA, SPL-40205), and flow cytometry staining was performed. That is, to the cultured cells washed with FACS buffer (PBS pH 7.4, 0.5% BSA, 0.01% Sodium Azide), an FC-blocker (BioLegend, US, cat. no. 422302) was added and allowed to react for 15 minutes, after which staining was performed for 30 minutes with a Live/Dead staining reagent (Thermo Fisher, US, cat. No. 65-0864-18), an anti-mouse CD19 antibody (BioLegend, US, cat. no. 302216), and an anti-mouse CD86 antibody (BD, US, cat. No. 560357). Thereafter, the cells were washed with FACS buffer and detected with a BD FACS Canto II.

As a result, it was confirmed that the 8G1-35-31 antibody can very effectively block the activation and proliferation of human B cells (FIG. 6 and FIG. 7).

### 3-6: Assay of the Non-Agonistic Property of the 8G1-35-31 Antibody

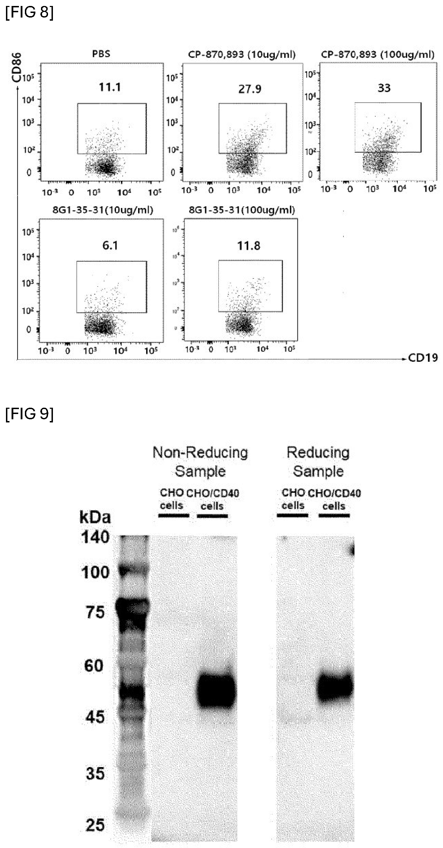

To confirm the effect of the drug itself on CD40-mediated signaling on the surface of immune cells, the following experiment was performed. Peripheral blood mononuclear cells (PBMCs) from two humans were treated with the 8G1-35-31 antibody at a concentration of 100 µg/ml and cultured in a 37°C, humidified, 5% CO₂ incubator for 3 days. As the positive control, CP870,893 (Pfizer) was treated at the same concentration, and as the negative control, a group not treated with antibody was used. After 3 days, the cultured cells were transferred to a FACS tube (USA, SPL-40205), and flow cytometry staining was performed. That is, to the cultured cells washed with FACS buffer (PBS pH 7.4, 0.5% BSA, 0.01% Sodium Azide), an FC-blocker (BioLegend, USA, cat. no. 422302) was added and allowed to react for 15 minutes, after which staining was performed for 30 minutes with a Live/Dead staining reagent (Thermo Fisher, USA, cat. No. 65-0864-18), an anti-mouse CD19 antibody (BioLegend, USA, cat. no. 302216), and an anti-mouse CD86 antibody (BD, USA, cat. No. 560357). The samples were detected with a BD FACS Canto II. As a result of the experiment, it was confirmed that the 8G1-35-31 antibody has a non-agonistic property of not activating human PBMCs on its own (FIG. 8).

### 3-7: Analysis of the Property of Recognizing the CD40 Sequence

To investigate whether the 8G1-35-31 antibody recognizes a linear antigen, Western blot analysis was performed. That is, CD40-CHO cells were washed once or twice with DPBS and lysed on ice for 20 minutes with 1× RIPA buffer (Biosesang, cat. No. R2002). Thereafter, the protein concentration of the supernatant was quantified using a BCA assay kit (Thermo Fisher Scientific cat. No. 2161296), and 2-mercaptoethanol was added to 1× Bolt^{™} LDS sample buffer to prepare samples under non-reducing and reducing conditions. The samples were denatured at 100°C for 5 minutes, then separated on a Bolt^{™} 4 to 12% Bis-Tris Gel, and transferred to a membrane. To confirm the transfer effect, the gel was stained with Coomassie Blue, and the membrane was checked with Ponceau S for 1 to 2 minutes; the membrane was then washed with 1× TBST and incubated with the 8G1-35-31 antibody (5 µg/ml) at 4°C for 18 hours with agitation. After incubation for 1 hour with the secondary antibody anti-mouse IgG-HRP, it was reacted with an ECL substrate (Thermo Scientific, cat. No. 32106), and the bands were confirmed with a ChemiDoc (BIO-RAD, US). As a result, it was confirmed that the 8G1-35-31 antibody can bind to linear CD40 (FIG. 9).

### 3-8: Investigation of Interspecies Cross-Binding Ability

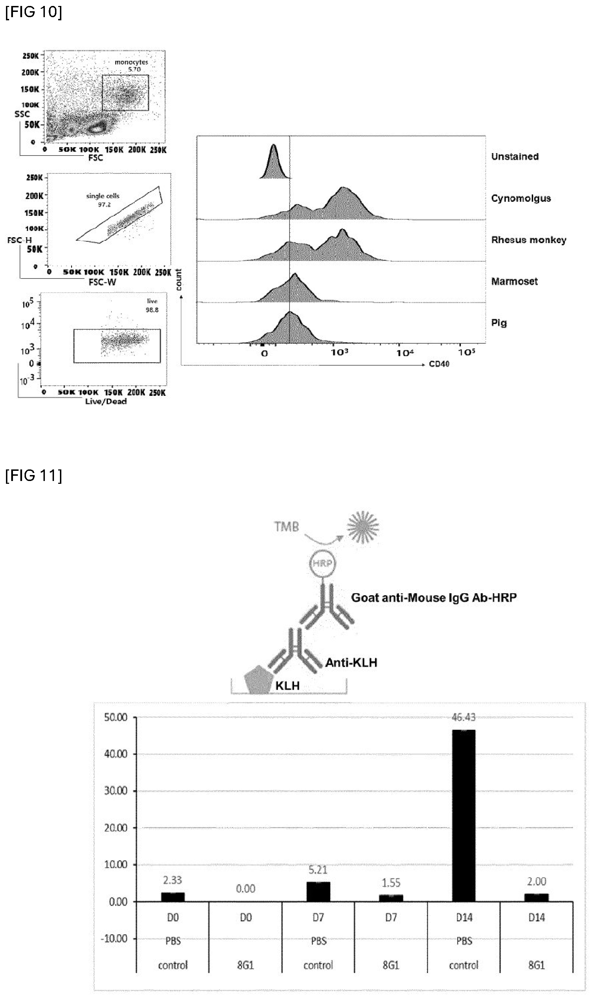

To confirm whether the 8G1-35-31 antibody binds to species other than human blood cells, flow cytometry was performed using PBMCs from primate cynomolgus, rhesus monkey, marmoset, and pig. Zombie Aqua (Biolegend) was applied to each cell and allowed to react at room temperature for 30 minutes, after which it was washed once with FACS buffer. Thereafter, the 8G1-35-31-FITC antibody (10 µg/ml) was applied and allowed to react on ice for 30 minutes. After washing twice with FACS buffer, analysis was performed using a BD LSR II. As a result, it was confirmed that the 8G1-35-31 antibody has interspecies cross-binding not only to humans but also to cynomolgus and rhesus monkeys (FIG. 10).

### 3-9: Antigen-Specific Antibody Production Inhibitory Effect of B Cells in hCD40/hCD40L-Expressing Mice

The hCD40/hCD40L double knock-in mice, technology-transferred from Model Organism (China), are mice in which human CD40 and CD40L are expressed, and in which mouse CD40 and mouse CD40L are gene-substituted with human CD40 and CD40L. These mice (9 weeks old) were intraperitoneally treated with 10 mg/kg KLH (Sigma), and the next day, the control group was treated with PBS and the experimental group was intraperitoneally treated with the 8G1-35-31 antibody (50 mg/kg). One week later, the control group was treated with KLH (10 mg/kg) and PBS (100 µl), and the experimental group was treated with KLH (10 mg/kg) and the 8G1-35-31 antibody (50 mg/kg). One week later, plasma was separated from peripheral blood, and the production of anti-KLH antibodies was analyzed by ELISA. That is, KLH (100 µg)/PBS 100 µl was placed in an ELISA plate and coated at 4°C for 18 hr. The next day, it was washed with 1× PBST (PBS, Tween, Thermo Fisher Scientific) and reacted with blocking buffer (Super Block, Thermo Fisher Scientific) at 37°C for 20 minutes. After washing three times again with PBST, 100 µl of an anti-KLH antibody standard solution (0-2,000 ng/ml) and a plasma dilution (1:1,000) was added and allowed to react at 37°C for 1 hour. After washing three times again with PBST, 100 µl of a goat anti-mouse IgG (FC-specific)-HRP antibody dilution (1:2,000) was added and allowed to react at 37°C for 1 hour, and the reaction was stopped with a stop solution. 50 µl each of TMB substrate (Thermo Fisher Scientific) was added and allowed to react at room temperature for 10 minutes, and the OD (optical density) value was measured with a fluorometer (450 nm, Thermo Fisher Scientific). As a result, it was confirmed that the 8G1-35-31 antibody can fundamentally block the production of anti-KLH antibodies in a KLH-immunized mouse model. Thereby, it was confirmed through an animal model that the 8G1-35-31 antibody can antigen-specifically inhibit the activation and proliferation of B cells in vivo (FIG. 11).

### 3-10: IFN-γ-Secreting Th1-Cell Activation Inhibitory Effect in hCD40/hCD40L-Expressing Mice

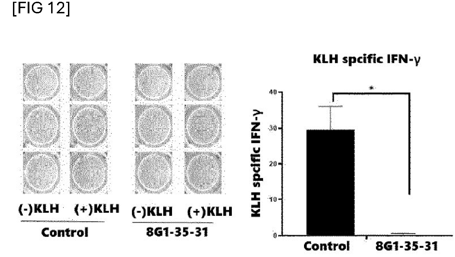

hCD40/hCD40L-expressing mice (9 weeks old) were intraperitoneally treated with 10 mg/kg KLH (Sigma), and the next day, the control group was treated with PBS and the experimental group was intraperitoneally treated with the 8G1-35-31 antibody (50 mg/kg). One week later, the control group was treated with KLH (10 mg/kg) and PBS (100 µl), and the experimental group was treated with KLH (10 mg/kg) and the 8G1-35-31 antibody (50 mg/kg). One week later, splenocytes were obtained from the spleens of the mice, and the production of KLH-specific Th1 cells was confirmed by ELISPOT (Enzyme-Linked ImmunoSpot) analysis. That is, an anti-IFN-γ capture antibody (15 µg/ml) was placed in an ELISPOT plate and coated at 4°C for 18 hours. The next day, after washing three times with phosphate-buffered saline (PBS, pH 7.4), the splenocytes of the control group and the experimental group were placed at a concentration of 1×10⁶, KLH antigen (100 ng/ml) was applied, and the cells were allowed to react at 37°C, 5% CO₂ for 24 hours. The cultured plate was taken out and washed three times with PBST, after which a detection antibody (biotin-conjugated) dilution (1:10,000) was added and allowed to react at room temperature for 2 hours. After washing three times with PBST, 100 µl of a streptavidin-HRP (1:1,000) dilution was applied and allowed to react at room temperature for 1 hour. After washing three times again with PBST, 100 µl of TMB substrate was applied and allowed to react for 20 minutes, after which it was washed with running water. The ELISPOT panel was dried at room temperature and then read with an ELISPOT reader. As a result, it was confirmed that 8G1-35-31 inhibits the activation and proliferation of KLH-antigen-specific Th1 cells in vivo (FIG. 12).

The foregoing description of the present invention is for illustration, and it is apparent to those of ordinary skill in the art to which the present invention pertains that various modifications and changes are possible without departing from the technical spirit of the present invention. The scope of the present invention is indicated by the claims set forth below rather than by the foregoing detailed description, and all changes or modifications derived from the meaning and scope of the claims and their equivalents should be construed as being included in the scope of the present invention.

### [INDUSTRIAL APPLICABILITY]

The novel anti-CD40 antibody of the present invention blocks CD40-CD40L signaling to exhibit an excellent antagonistic effect, and thus is expected to be widely utilized as an agent for preventing or treating various diseases, such as autoimmune diseases and chronic inflammatory diseases, and as an agent for suppressing immune rejection upon transplantation, and therefore has industrial applicability.
Name of depositary institution: Korean Cell Line Research Foundation
Accession number: KCLRF-BP-00523
Date of deposit: June 1, 2023

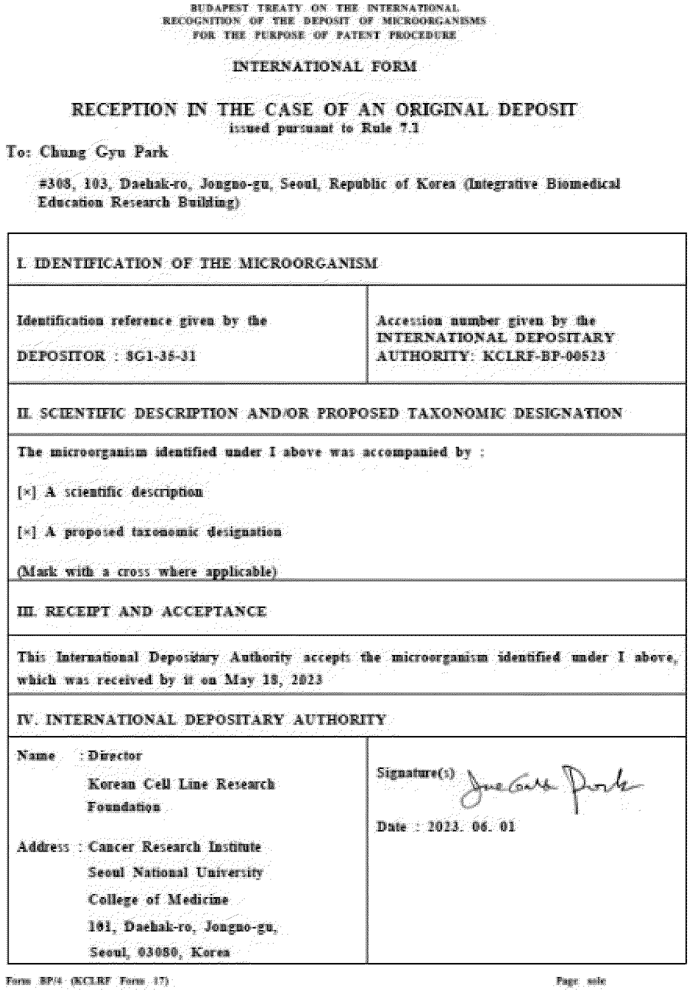
BUDAPEST TREATY ON THE INTERNATIONAL
RECOGNITION OF THE DEPOSIT OF MICROORGANISMS
FOR THE PURPOSE OF PATENT PROCEDURE

INTERNATIONAL FORM

RECEPTION IN THE CASE OF AN ORIGINAL DEPOSIT
issued pursuant to Rule 7.1

To: Chung Gyu Park

#308, 103, Daehak-ro, Jongno-gu, Seoul, Republic of Korea (Integrative Biomedical
Education Research Building)

I. IDENTIFICATION OF THE MICROORGANISM

Identification reference given by the
DEPOSITOR : SG1-35-31 | Accession number given by the
INTERNATIONAL DEPOSITARY
AUTHORITY: KCLRF-BP-00523

II. SCIENTIFIC DESCRIPTION AND/OR PROPOSED TAXONOMIC DESIGNATION

The microorganism identified under I above was accompanied by :

[×] A scientific description

[×] A proposed taxonomic designation

(Mark with a cross where applicable)

III. RECEIPT AND ACCEPTANCE

This International Depositary Authority accepts the microorganism identified under I above,
which was received by it on May 18, 2023

IV. INTERNATIONAL DEPOSITARY AUTHORITY

Name : Director
Korean Cell Line Research
Foundation

Address : Cancer Research Institute
Seoul National University
College of Medicine
101, Daehak-ro, Jongno-gu,
Seoul, 03080, Korea | Signature(s)

Date : 2023. 06. 01

Form BP/4 (KCLRF Form 17) Page: sole

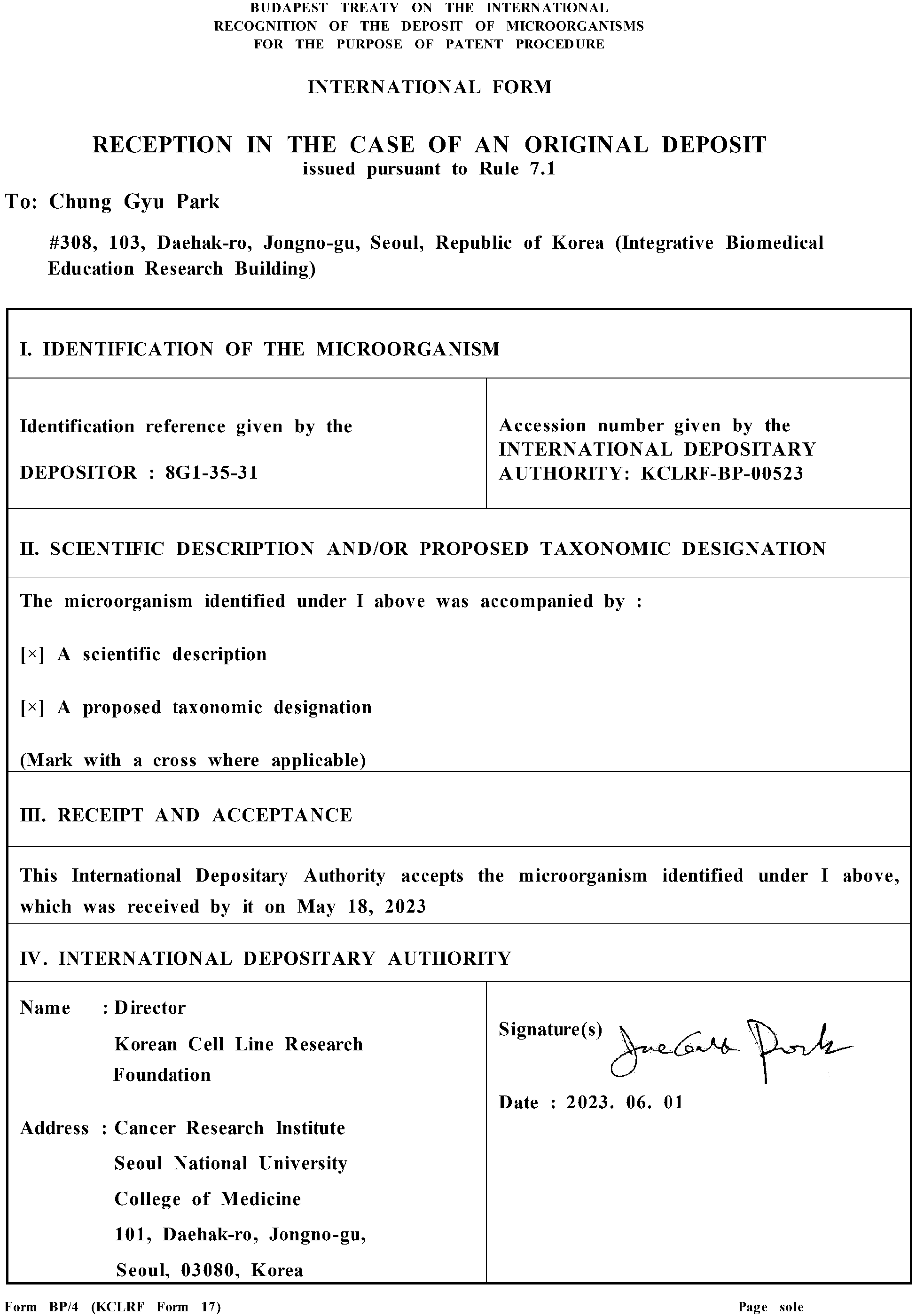
BUDAPEST TREATY ON THE INTERNATIONAL
RECOGNITION OF THE DEPOSIT OF MICROORGANISMS
FOR THE PURPOSE OF PATENT PROCEDURE

INTERNATIONAL FORM

RECEPTION IN THE CASE OF AN ORIGINAL DEPOSIT
issued pursuant to Rule 7.1

To: Chung Gyu Park

#308, 103, Daehak-ro, Jongno-gu, Seoul, Republic of Korea (Integrative Biomedical
Education Research Building)

I. IDENTIFICATION OF THE MICROORGANISM

Identification reference given by the
DEPOSITOR : 8G1-35-31 | Accession number given by the
INTERNATIONAL DEPOSITARY
AUTHORITY: KCLRF-BP-00523

II. SCIENTIFIC DESCRIPTION AND/OR PROPOSED TAXONOMIC DESIGNATION

The microorganism identified under I above was accompanied by :

[×] A scientific description

[×] A proposed taxonomic designation

(Mark with a cross where applicable)

III. RECEIPT AND ACCEPTANCE

This International Depositary Authority accepts the microorganism identified under I above,
which was received by it on May 18, 2023

IV. INTERNATIONAL DEPOSITARY AUTHORITY

Name : Director
Korean Cell Line Research
Foundation

Address : Cancer Research Institute
Seoul National University
College of Medicine
101, Daehak-ro, Jongno-gu,
Seoul, 03080, Korea | Signature(s)

Date : 2023. 06. 01

Form BP/4 (KCLRF Form 17) Page sole

## Claims

1. An anti-CD40 antibody or an antigen-binding fragment thereof comprising the following complementarity determining regions (CDRs):
(i) an HCDR1 having the amino acid sequence of SEQ ID NO: 1 or a sequence having at least 80% sequence identity thereto;
(ii) an HCDR2 having the amino acid sequence of SEQ ID NO: 2 or a sequence having at least 80% sequence identity thereto;
(iii) an HCDR3 having the amino acid sequence of SEQ ID NO: 3 or a sequence having at least 80% sequence identity thereto;
(iv) an LCDR1 having the amino acid sequence of SEQ ID NO: 4 or a sequence having at least 80% sequence identity thereto;
(v) an LCDR2 having the amino acid sequence of SEQ ID NO: 5 or a sequence having at least 80% sequence identity thereto; and
(vi) an LCDR3 having the amino acid sequence of SEQ ID NO: 6 or a sequence having at least 80% sequence identity thereto.

2. The anti-CD40 antibody or antigen-binding fragment thereof according to claim 1, wherein the anti-CD40 antibody comprises a heavy chain variable region having the amino acid sequence of SEQ ID NO: 13 or a sequence having at least 80% sequence identity thereto, and a light chain variable region having the amino acid sequence of SEQ ID NO: 14 or a sequence having at least 80% sequence identity thereto.

3. The anti-CD40 antibody or antigen-binding fragment thereof according to claim 1, wherein the anti-CD40 antibody or antigen-binding fragment thereof is produced by a hybridoma cell line deposited under accession number KCLRF-BP-00523.

4. The anti-CD40 antibody or antigen-binding fragment thereof according to claim 1, wherein the anti-CD40 antibody is selected from the group consisting of a monoclonal antibody, a chimeric antibody, a primatized antibody, a humanized antibody, and a human antibody.

5. The anti-CD40 antibody or antigen-binding fragment thereof according to claim 1, wherein the anti-CD40 antibody is selected from the group consisting of a multimeric antibody, a heterodimeric antibody, a half antibody, a tetravalent antibody, a bispecific antibody, and a single-chain antibody.

6. The anti-CD40 antibody or antigen-binding fragment thereof according to claim 1, wherein the antigen-binding fragment is selected from the group consisting of Fab, F(ab)2, Fab', F(ab)2, F(ab')3, Fd, Fv, and a domain antibody.

7. A nucleic acid encoding the anti-CD40 antibody according to any one of claims 1 to 6.

8. A vector comprising the nucleic acid according to claim 7.

9. A host cell comprising the nucleic acid according to claim 7.

10. A method for producing an antibody, comprising culturing the host cell according to claim 9 to express the antibody.

11. An antibody-drug conjugate comprising, as an active ingredient, the anti-CD40 antibody according to any one of claims 1 to 6.

12. The antibody-drug conjugate according to claim 11, wherein the drug is selected from the group consisting of an anticancer agent, an aptamer, an antisense oligonucleotide, a small interfering RNA (siRNA), and a microRNA.
